(19) Europäisches Patentamt
European Patent Office
Office européen des brevets

(11) Publication number: **0 000 151**
**B1**

(12) **EUROPEAN PATENT SPECIFICATION**

(45) Date of publication of patent specification: **20.05.81**

(51) Int. Cl.³: **C 07 D 403/12**, A 61 K 31/415

(21) Application number: **78100166.4**

(22) Date of filing: **15.06.78**

(54) **1-Substituted aminoindolines, process for their production and pharmaceutical compositions containing them.**

(30) Priority: **28.06.77 CH 7915/77**

(43) Date of publication of application: **10.01.79 Bulletin 79/1**

(45) Publication of the grant of the patent: **20.05.81 Bulletin 81/20**

(84) Designated Contracting States: **BE CH DE FR GB LU NL SE**

(56) References cited:
**CH A 529 702**
**FR A 2 230 363**

(73) Proprietor: **SANDOZ AG, Lichtstrasse 35, CH-4002 Basel (CH)**

(72) Inventor: **Bormann, Gerhard, Dr., Grosse Allee 88, CH-4142 Münchenstein (CH)**
Inventor: **Berthold, Richard, Dr., Ahornstrasse 9, CH-4103 Bottmingen (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



BUNDESDRUCKEREI BERLIN

# 1-substituted aminoindolines, process for their production and pharmaceutical compositions containing them

The present invention relates to 1-substituted aminoindoline derivatives.
In accordance with the invention there are provided compounds of formula I,

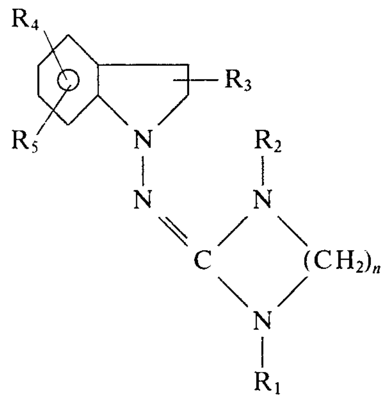

(I)

wherein

one of $R_1$ and $R_2$ is hydrogen and the other is hydrogen or alkyl of 1 to 4 carbon atoms,
$R_3$ is hydrogen or alkyl of 1 to 4 carbon atoms, in the 2- or 3-position,
$R_4$ and $R_5$ independently are hydrogen, alkyl of 1 to 4 carbon atoms, alkoxy of 1 to 4 carbon atoms, alkylthio of 1 to 4 carbon atoms or halogen of atomic number of from 9 to 35, and
n is 2, 3 or 4.

Alkyl, alkoxy and alkylthio preferably contain 1 or 2, especially 1 carbon atom. Halogen is preferably chlorine.

$R_1$, $R_2$, $R_3$ and $R_5$ are preferably hydrogen. $R_4$ is preferably hydrogen, alkyl or halogen, especially hydrogen.

$R_3$ is preferably in the 3 position of the indoline nucleus.
$R_4$ is preferably in the 4, 5 or 6, especially in the 4 or 5, preferably in the 4-position.
n is preferably 2.

In accordance with the invention, a compound of formula I may be obtained by a process comprising cyclizing a compound of formula II,

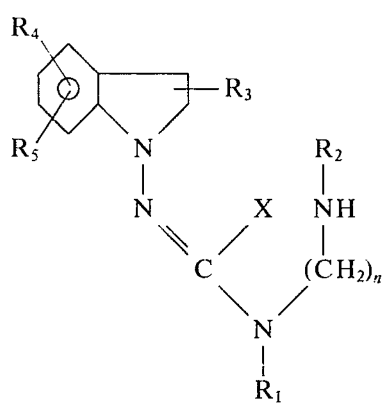

(II)

wherein

$R_1$ to $R_5$ and n are as defined above and
X is a leaving group.

The process according to the invention may be effected in a manner analogous to known methods for cyclizing analogous amino derivatives. X is e. g. a group $-NHR_a$, wherein $R_a$ is alkyl of 1 to 4 carbon

atoms, especially methyl, or $R_a$ is hydrogen. The reaction is preferably effected in an inert solvent such as methanol or ethanol, or, when the amine of formula IV (see below) is liquid at the reaction temperature, the reaction is conveniently effected in the absence of any additional solvent. The reaction is preferably effected in the presence of a mineral acid such as hydrochloric or hydroiodic acid. The reaction temperature may be from room temperature to about 150° C and is preferably at least 50° C, e. g. the boiling temperature of the reaction mixture.

The compounds of formula I may be isolated and purified in accordance with known methods.

The compounds of formula I may be present in free form, or in the form of acid addition salts. Acid addition salt forms, for example, the hydrochloride or hydrogen maleate, may be produced from the free form in known manner, and vice-versa.

The compounds of formula I may also be present in tautomeric form, i. e. with the double bond adjacent to one of the other two nitrogen atoms of the guanidine moiety, insofar as this nitrogen atom is not substituted by an alkyl group $R_1$ or $R_2$. It is to be appreciated that such tautomeric forms also fall under the scope of formula I.

The production of the starting materials may be effected in known manner.

A compound of formula II may e. g. be produced by reacting a compound of formula III,

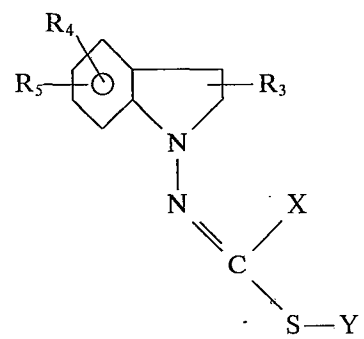

(III)

wherein $R_3$ to $R_5$ and X are as defined above and the group $-S-Y$ is a leaving group, with a compound of formula IV,

$$R_1-NH-(CH_2)_n-NH-R_2 \qquad (IV)$$

wherein $R_1$, $R_2$ and n are as defined above.

Y may e. g. be alkyl of 1 to 4 carbon atoms, preferably methyl. The reaction conditions may be chosen such as to be identical with the conditions for cyclization according to the invention. The compounds of formula III are then advantageously reacted with the compounds of formula IV to give directly the corresponding compounds of formula I, without intermediate isolation of the compounds of formula II.

When in the compounds of formula III Y is alkyl of 1 to 4 carbon atoms and X is a group $-NH-R_a$, the reaction conditions for producing compounds of formula I directly from corresponding compounds of formula III are analogous to known reaction conditions for the production of a 1-(indolin-1-yl)-guanidine derivative from a 1-(indolin-1-yl)-2-(lower)alkylisothiourea.

Insofar as the production of the starting materials is not described, these are known or may be produced and purified in accordance with known processes, or in a manner analogous to the processes described above or analogous to known processes.

In the following non-limitative Examples all temperatures are indicated in degrees Centigrade and are uncorrected.

### Example 1
### 1-(Imidazolidin-2-ylidenamino)indoline

To 10 g 1-(indolin-1-yl)-2-methylisothiourea hydrochloride dissolved in 40 ml ethanol are added 8 ml ethylene diamine and the reaction mixture is boiled for 6 hours with stirring. The mixture is then evaporated to dryness and the residue is extracted from a 1 M solution of sodium hydroxide with methylene chloride. The organic phase is then dried over magnesium sulphate and evaporated. The title compound is obtained (M. P. of the hydrogen maleate 171—172° — from ethanol/ether).

The starting material is obtained as follows:

1-Aminoindoline is reacted with benzoyl isothiocyanate in boiling tetrahydrofurane and, after saponification of the product over 15 minutes with diluted sodium hydroxide under reflux, 1-(indolin-1-yl)thiourea (M. P. 225—227° — from methanol) is obtained. This product is converted into 1-(indolin-1-yl)-2-methylisothiourea hydroiodide by heating up for 1 hour with methyl iodide in methanol. The free base is obtained by addition of aqueous sodium hydroxide and is further reacted

with a 2N methanolic solution of hydrochloric acid to give 1-(indolin-1-yl)-2-methylisothiourea hydrochloride (M. P. 227—229° — from methanol/ether).

The following compounds of formula I are obtained in a manner analogous to Example 1, using the corresponding starting materials of formula III, wherein Y is methyl and X is $-NH_2$ or $-NH-CH_3$, and of formula IV:

| Ex. No. | $R_1$ | $R_2$ | $R_3$ | $R_4$ | $R_5$ | n | M. P. |
|---|---|---|---|---|---|---|---|
| 2 | H | H | H | 5-Cl | H | 2 | b 182—184° |
| 3 | H | H | 2-Me | H | H | 2 | hcl 226—228° |
| 4 | H | H | 3-Me | H | H | 2 | b 173—174° |
| 5 | H | H | H | 4-Me | H | 2 | b 148—149° |
| 6 | H | H | H | 4-OMe | H | 2 | hma 160—162° |
| 7 | H | H | H | 7-Me | H | 2 | hcl 194—196° |

b = in free form

hcl = hydrochloride

hma = hydrogen maleate

The compounds of formula I exhibit pharmacological activity. In particular, the compounds possess vasoconstricting activity, as indicated by standard tests. For example, this activity may be observed in vivo in rats treated in accordance with the principles of J. S. Gillespie and T. C. Muir, Br. J. Pharmac. Chemother. (1967) 30, 78—87): a pressor effect is elicited following i. v. administration of from about 0.02 to about 50 µg/kg, particularly of from about 0.02 to about 0.5 µg/kg of the compounds.

The compounds are therefore indicated for use as vasoconstricting agents, e. g. for the prophylaxis and treatment of vascular headaches such as migraine, and of orthostatic disorders such as orthostatic hypotension and its symptoms, such as vertigo.

For this use an indicated daily dose is from about 0.0025 to about 1 mg, conveniently administered in divided doses 2 to 4 times a day in unit dosage form containing from about 0.0005 to about 0.5 mg, or in sustained release form.

The activity of the compound of Example 1 is especially interesting.

The compounds of formula I may be administered in free form or in pharmaceutically acceptable acid addition salt form. Such forms exhibit the same order of activity as the free form. The present invention also provides a pharmaceutical composition comprising a compound of formula I, in free form or in pharmaceutically acceptable salt form, in association with a pharmaceutical carrier or diluent. Such compositions may be in the form of, for example, a solution or a tablet.

## Claims

1. A process for the production of a compound of formula I,

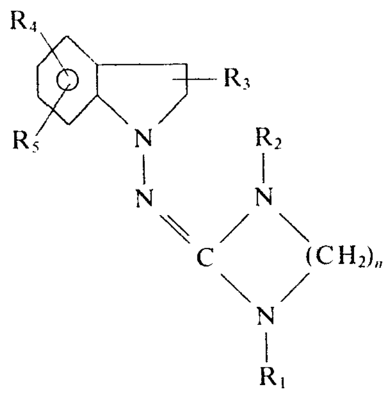

(I)

wherein

one of $R_1$ and $R_2$ is hydrogen and the other is hydrogen or alkyl of 1 to 4 carbon atoms,
$R_3$ is hydrogen or alkyl of 1 to 4 carbon atoms, in the 2- or 3-position,
$R_4$ and $R_5$ independently are hydrogen, alkyl of 1 to 4 carbon atoms, alkoxy of 1 to 4 carbon atoms, alkylthio of 1 to 4 carbon atoms or halogen of atomic number of from 9 to 35, and
n is 2, 3 or 4,

or a tautomeric form thereof, which comprises cyclizing a compound of formula II,

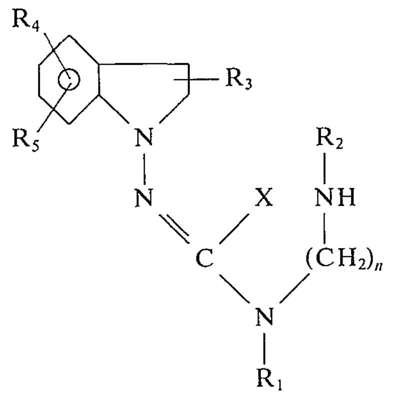

(II)

wherein

$R_1$ to $R_5$ and n are as defined above and
X is a leaving group.

2. A compound of formula I, as defined in claim 1, or a tautomeric form thereof.
3. A compound of claim 2, wherein $R_1$ to $R_5$ are hydrogen and n is 2.
4. A compound of claim 2, wherein $R_1$, $R_2$ and $R_5$ are hydrogen and n is 2.
5. A compound of claim 4, wherein $R_3$ is hydrogen and $R_4$ is 5-chloro, or $R_3$ is 2-methyl and $R_4$ is hydrogen.
6. A compound of claim 4, wherein $R_3$ is 3-methyl and $R_4$ is hydrogen, or $R_3$ is hydrogen and $R_4$ is 4-methyl.
7. A compound of claim 4, wherein $R_3$ is hydrogen and $R_4$ is 4-methoxy, or $R_3$ is hydrogen and $R_4$ is 7-methyl.
8. A compound according to any one of claims 2 to 7, in free form.
9. A compound according to any one of claims 2 to 7, in acid addition salt form.
10. A pharmaceutical composition comprising a compound according to any one of claims 2 to 7 in free form or in pharmaceutically acceptable acid addition salt form, in association with a pharmaceutical carrier or diluent.
11. A compound of claim 2, for use in a method for treatment of the human or animal body by therapy.

**Patentansprüche**

1. Ein Verfahren zur Herstellung einer Verbindung der Formel I,

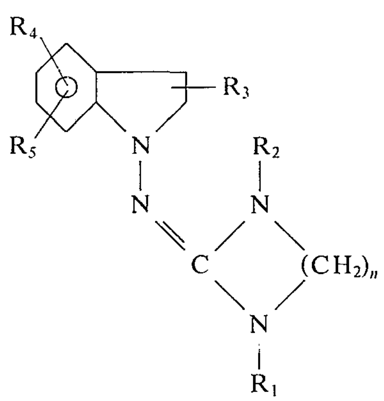

(I)

worin

eines von $R_1$ und $R_2$ Wasserstoff und das andere Wasserstoff oder Alkyl mit 1—4 Kohlenstoffatomen bedeutet,

$R_3$ Wasserstoff oder in 2- oder 3-Stellung ständiges Alkyl mit 1—4 Kohlenstoffatomen bedeutet,

$R_4$ und $R_5$ unabhängig voneinander für Wasserstoff, Alkyl mit 1—4 Kohlenstoffatomen, Alkoxy mit 1—4 Kohlenstoffatomen, Alkylthio mit 1—4 Kohlenstoffatomen oder Halogen mit einer Ordnungszahl von 9 bis 35 stehen, und

n für die Zahl 2, 3 oder 4 steht,

oder einer ihrer tautomeren Formen, beinhaltend die Cyclisierung einer Verbindung der Formel II,

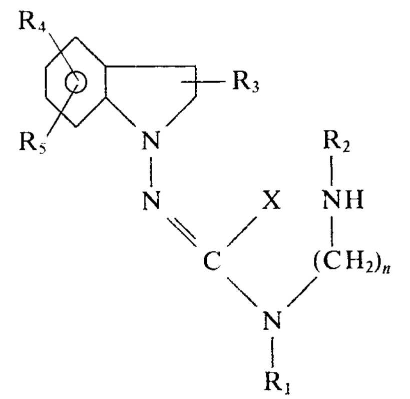

(II)

worin

$R_1$ bis $R_5$ und n obige Bedeutung besitzen und

X für eine Abgangsgruppe steht.

2. Eine Verbindung der Formel I, wie in Anspruch 1 definiert, oder eine ihrer tautomeren Formen.

3. Eine wie in Anspruch 2 definierte Verbindung, in der $R_1$ bis $R_5$ Wasserstoff bedeuten und n für die Zahl 2 steht.

4. Eine wie in Anspruch 2 definierte Verbindung, in der $R_1$, $R_2$ und $R_5$ Wasserstoff bedeuten und n für die Zahl 2 steht.

5. Eine wie in Anspruch 4 definierte Verbindung, in der $R_3$ Wasserstoff bedeutet und $R_4$ für 5-Chlor steht, oder $R_3$ 2-Methyl bedeutet und $R_4$ für Wasserstoff steht.

6. Eine wie in Anspruch 4 definierte Verbindung, in der $R_3$ 3-Methyl bedeutet und $R_4$ für Wasserstoff steht, oder $R_3$ Wasserstoff bedeutet und $R_4$ für 4-Methyl steht.

7. Eine wie in Anspruch 4 definierte Verbindung, in der $R_3$ Wasserstoff bedeutet und $R_4$ für 4-Methoxy steht, oder $R_3$ Wasserstoff bedeutet und $R_4$ für 7-Methyl steht.

8. Eine Verbindung, wie in irgendeinem der Ansprüche 2 bis 7 definiert, in freier Form.

9. Eine Verbindung, wie in irgendeinem der Ansprüche 2 bis 7 definiert, in Säureadditionssalzform.

10. Eine pharmazeutische Zusammensetzung, enthaltend eine Verbindung, wie in irgendeinem der Ansprüche 2 bis 7 definiert, in freier Form oder in physiologisch verträglicher Säureadditionssalzform, zusammen mit einem Träger- bzw. Verdünnungsmittel.

11. Eine Verbindung, wie in Anspruch 2 definiert, zur Anwendung in einem Verfahren zur therapeutischen Behandlung des menschlichen oder tierischen Körpers.

**Revendications**

1. Un procédé de préparation d'un composé de formule I

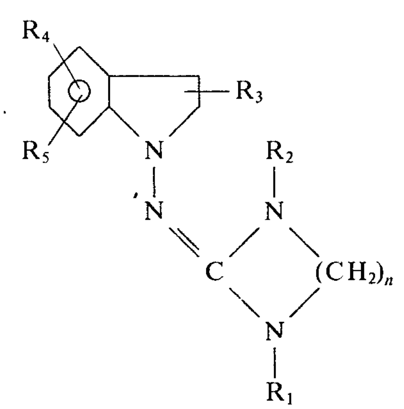

(I)

dans laquelle

l'un des symboles $R_1$ et $R_2$ est l'hydrogène et l'autre est l'hydrogène ou un groupe alkyle de 1 à 4 atomes de carbone,

$R_3$ est l'hydrogène ou un groupe alkyle de 1 à 4 atomes de carbone, en position 2 ou 3,

$R_4$ et $R_5$ signifient indépendamment l'hydrogène, un groupe alkyle de 1 à 4 atomes de carbone, alcoxy de 1 à 4 atomes de carbone, alkylthio de 1 à 4 atomes de carbone ou un halogène d'un nombre atomique compris entre 9 et 35, et

n signifie 2, 3 ou 4,

ou une forme tautomère de ce composé, comprenant la cyclisation d'un composé de formule II

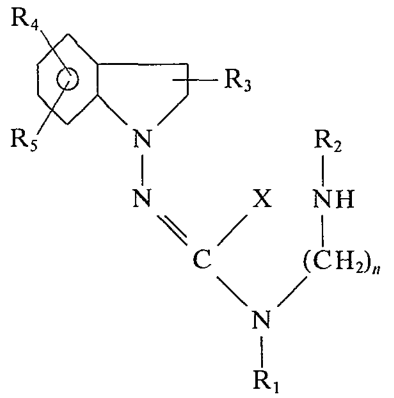

(II)

dans laquelle $R_1$ à $R_5$ et n sont tels que définis ci-dessus et X est un groupe susceptible d'être éliminé.

2. Un composé de formule I, tel que défini à la revendication 1, ou une forme tautomère de ce composé.

3. Un composé de la revendication 2, dans lequel $R_1$ à $R_5$ sont l'hydrogène et n est 2.

4. Un composé de la revendication 2, dans lequel $R_1$, $R_2$ et $R_5$ sont l'hydrogène et n est 2.

5. Un composé de la revendication 4, dans lequel $R_3$ est l'hydrogène et $R_4$ est un atome de chlore en position 5, ou $R_3$ est un groupe méthyle en position 2 et $R_4$ est l'hydrogéne.

6. Un composé de la revendication 4, dans lequel $R_3$ est un groupe méthyle en position 3 et $R_4$ est l'hydrogène, ou $R_3$ est l'hydrogène et $R_4$ est un groupe méthyle en position 4.

7. Un composé de la revendication 4, dans lequel $R_3$ est l'hydrogène et $R_4$ est un groupe méthoxy en position 4, ou $R_3$ est l'hydrogène et $R_4$ est un groupe méthyle en position 7.

8. Un composé selon l'une quelconque des revendications 2 à 7, sous forme libre.

9. Un composé selon l'une quelconque des revendications 2 à 7, sous forme de sel d'addition d'acide.

10. Une composition pharmaceutique comprenant un composé selon l'une quelconque des revendications 2 à 7 sous forme libre ou sous forme d'un sel d'addition d'acide acceptable du point de vue pharmaceutique, en association avec un excipient ou diluant pharmaceutique.

11. Un composé de la revendication 2, pour l'utilisation dans une méthode de traitement thérapeutique du corps humain ou animal.